(19) Europäisches Patentamt
European Patent Office
Office européen des brevets

(11) **EP 3 683 202 B1**

(12) **EUROPÄISCHE PATENTSCHRIFT**

(45) Veröffentlichungstag und Bekanntmachung des Hinweises auf die Patenterteilung:
**26.04.2023   Patentblatt 2023/17**

(21) Anmeldenummer: **19020035.2**

(22) Anmeldetag: **18.01.2019**

(51) Internationale Patentklassifikation (IPC):
**C07C 1/20** (2006.01)   **C07C 11/06** (2006.01)
**C10G 3/00** (2006.01)   **C07C 4/06** (2006.01)
**C07C 7/00** (2006.01)   **C07C 7/09** (2006.01)

(52) Gemeinsame Patentklassifikation (CPC):
(C-Sets verfügbar)
**C07C 1/20; C07C 4/06; C07C 7/005; C07C 7/09; C10G 3/42;** C10G 2400/02; C10G 2400/20; C10G 2400/22; Y02P 30/20; Y02P 30/40   (Forts.)

(54) **VERFAHREN ZUR HERSTELLUNG VON OLEFINEN AUS OXYGENATEN**

PROCESS FOR PRODUCING OLEFINS FROM OXYGENATES

PROCÉDÉ DE PRODUCTION D'OLÉFINES À PARTIR DE COMPOSÉS OXYGÉNÉS

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(43) Veröffentlichungstag der Anmeldung:
**22.07.2020   Patentblatt 2020/30**

(73) Patentinhaber: **L'AIR LIQUIDE, SOCIÉTÉ ANONYME POUR L'ÉTUDE ET L'EXPLOITATION DES PROCÉDÉS GEORGES CLAUDE**
**75007 Paris (FR)**

(72) Erfinder:
• **Ahlers, Bernd**
  **63128 Dietzenbach (DE)**
• **Castillo-Welter, Frank**
  **61381 Friedrichsdorf (DE)**
• **Drosdzol, Christopher**
  **60438 Frankfurt (DE)**
• **Gorny, Martin**
  **65760 Eschborn (DE)**
• **Haag, Stéphane**
  **60598 Frankfurt (DE)**

• **Janko, Lutz**
  **64390 Erzhausen (DE)**
• **Williams, Bryce**
  **60437 Frankfurt am Main (DE)**

(74) Vertreter: **Dropsch, Holger**
**Air Liquide Forschung und Entwicklung GmbH**
**Gwinnerstraße 27-33**
**60388 Frankfurt am Main (DE)**

(56) Entgegenhaltungen:
**DE-A1-102013 101 575**

(52) Gemeinsame Patentklassifikation (CPC): (Forts.)

C-Sets
**C07C 1/20, C07C 11/02;**
**C07C 1/20, C07C 11/06;**
**C07C 4/06, C07C 11/02;**
**C07C 4/06, C07C 11/06;**
**C07C 7/005, C07C 11/02;**
**C07C 7/005, C07C 11/06;**
**C07C 7/09, C07C 11/02;**
**C07C 7/09, C07C 11/06**

## Beschreibung

In einem Verfahren zur Herstellung von Olefinen aus Oxygenaten, umfassend die folgenden Schritte:

(i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,
(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird, betrifft die Erfindung gemäss Ansprüche die weitere Aufarbeitung der erhaltenen Ströme zur Gewinnung insbesondere kurzkettiger Olefine wie Ethylen und Propylen. Speziell betrifft die Erfindung die Weiterverarbeitung oxygenathaltiger Ströme im Rahmen dieser Aufarbeitung.

## Stand der Technik

Kurzkettige Olefine, insbesondere Propylen (Propen), gehören zu den wichtigsten Grundstoffen der chemischen Industrie. Dies liegt darin begründet, das ausgehend von diesen ungesättigten Verbindungen mit einer kurzen Kettenlänge Moleküle mit langkettigem Kohlenstoffgerüst und zusätzlichen Funktionalisierungen aufgebaut werden können.

Als Quelle für kurzkettige Olefine diente in der Vergangenheit vor allem das Steam-Cracking, d.h. die thermische Spaltung bei der Erdölverarbeitung. In den vergangenen Jahren wurden jedoch weitere Prozesse zur Herstellung von kurzkettigen Olefinen entwickelt. Dies ist zum einen durch steigende Nachfrage bedingt, die durch die vorhandenen Quellen nicht mehr gedeckt werden kann; zum anderen fordert die zunehmende Verknappung von fossilen Rohstoffen die Verwendung anderer Ausgangsstoffe.

Die sog. MTP- (Methanol-to-Propylen) oder auch MTO- (Methanol-to-Olefin) Verfahren zur Herstellung von Propylen und anderen kurzkettigen Olefinen gehen von Methanol als Ausgangsstoff aus. Verallgemeinernd spricht man in diesem Zusammenhang auch von Oxygenat-zu-Olefin-(OTO)-Verfahren, da sauerstoffhaltige organische Komponenten wie Methanol oder Dimethylether (DME) auch als Oxygenate bezeichnet werden. In diesen heterogen katalysierten Verfahren wird demnach beispielsweise aus Methanol zunächst teilweise das Zwischenprodukt Dimethylether und nachfolgend aus einer Mischung von Methanol und Dimethylether eine Mischung aus Ethylen und Propylen sowie Kohlenwasserstoffen mit höherer Molmasse, darunter auch Olefine, gebildet. Zudem findet sich im Produktstrom Wasser, welches zum einen aus dem Prozessdampf stammt, der optional dem MTO-Reaktor zur Reaktionsmodulierung zugeführt wird und dem im MTP-Reaktor erzeugten Reaktionswasser.

Die sich anschließende Aufreinigung soll zum einen unerwünschte Nebenprodukte und unumgesetzte Edukte abtrennen und die einzelnen Kohlenwasserstofffraktionen möglichst rein darstellen. Üblicherweise wird hierzu im ersten Schritt ein Quenchsystem zur Anwendung gebracht. Unter Quenchen wird dabei eine schlagartige oder schockartige Abkühlung verstanden, die zumeist durch direkten Wärmeaustausch mit einem fluiden Quenchmedium bewirkt wird. Wenn hierzu eine Flüssigkeit wie Wasser oder Methanol verwendet wird, tritt zudem eine gewisse Reinigungswirkung in Bezug auf die verbliebene Gasphase auf.

An den Quenchschritt schließen sich zumeist eine Kompressionssektion und schließlich eine Aufarbeitungssektion an, bei der die verschiedenen Kohlenwasserstoffe als Fraktionen oder auch - insbesondere die als Zielprodukte begehrten Olefine - als isolierte Komponenten erhalten werden. Die Aufarbeitungssektion besteht dabei in der Regel aus einer Abfolge verschalteter Destillations- bzw. Rektifikationskolonnen.

Ein Beispiel für die einer OTO-Reaktion nachfolgende Aufreinigung findet sich in der DE 10 2014 112 792 A1, in der beschrieben wird, wie in einem ersten Schritt eine heterogen-katalysierte Umsetzung von wenigstens einem Oxygenat zu einem C2 Olefine, C3 Olefine, C4 Olefine, C5/6 Kohlenwasserstoffverbindungen und C7+ Kohlenwasserstoffverbindungen enthaltenden Produktstrom und in einem zweiten Schritt eine Abtrennung eines zu wenigstens 95 Gew.-% aus C3 Olefinen bestehenden Propylenstroms erzeugt wird.

Die Patentveröffentlichung DE 102013101575 A1 lehrt ein Verfahren zur Herstellung von Olefinen aus Oxygenaten, umfassend die folgenden Schritte: (i) heterogen-katalysierte Umsetzung von wenigstens einem Oxygenat zu einem flüssige und gasförmige organische Verbindungen und Wasser enthaltenden Gesamtstrom und (ii) Auftrennung des Gesamtstroms in einer ersten Trenneinrichtung in eine zu wenigstens 90 Vol.-% die gasförmigen organischen Verbindungen des Gesamtstroms enthaltende Fraktion, in eine zu wenigstens 90 Gew.-% die flüssigen organischen Verbindungen des Gesamtstroms enthaltende Fraktion und in eine zu wenigstens 90 Gew.-% das Wasser des Gesamtstroms enthaltende Fraktion.

Die weiteren Aufreinigungseinheiten, die in der DE 10 2014 112 792 A1 beschrieben werden, entsprechen dem im Stand der Technik üblichen Konzept. Durch das Quenchen kann bereits eine grobe Trennung der Fraktionen in Abhängigkeit von ihrer Kettenlänge der entstehenden Olefine aufgrund des teilweisen Auskondensierens erfolgen, so dass eine flüssige C4+ Fraktion aus dem Quench abgeführt werden kann. Die gasförmig abgetrennte C4- Fraktion wird anschließend in eine Kompressionsstufe eingespeist. Die aus der Kompression stammende C4- Fraktion wird dann einer Trennvorrichtung zugeführt, in der C3- Kohlenwasserstoffe von den C4+ Kohlenwasserstoffen separiert werden. In anschließenden Reinigungsschritten wird die C3 Fraktion in einer weiteren Trenneinrichtung von der C2- Fraktion getrennt, was aufgrund der geringen Siedepunkte der beiden Fraktionen unter Druck erfolgen muss. Insgesamt ist die Aufreinigung des Produktstroms kompliziert, da eine hohe Reinheit der Produkte angestrebt wird. Dies gilt für MTP-Anlagen mit üblichen Produktionskapazitäten von etwa 470 kta Propylen, wird aber noch entscheidender, wenn die Anlagenkapazität geringer ist (100 kta oder 200 kta Propylen). Dadurch sind kleine Anlagen zurzeit wirtschaftlich nicht rentabel.

Bei der Aufarbeitung der als Reaktorprodukt erhaltenen Kohlenwasserstofffraktionen ist insbesondere die Abtrennung nicht umgesetzter Oxygenate, vor allem von DME, vor Bedeutung. Aufgrund seiner Siedepunktslage würde DME ansonsten vor allem das Propylen-Endprodukt kontaminieren. Da Propylen nachfolgend häufig einer Polymerisierung zugeführt werden soll und sauerstoffhaltige Bestandteile wie DME als Katalysatorgift auf die verwendeten Polymerisierungskatalysator wirken können, ist seine weitgehende Abscheidung aus den Reaktionsprodukten von großer Bedeutung. Erreicht wird diese weitgehende Abscheidung gemäß Stand der Technik durch eine Extraktivdestillation, bei der bevorzugt ein verfahrenseigener Stoff, z. B. Methanol, als Extraktionsmittel eingesetzt wird. Im Sumpf der Extraktivdestillationskolonne wird dann eine Kohlenwasserstofffraktion erhalten, die auch das abgetrennte Oxygenat wie DME sowie das Extraktionsmittel Methanol enthält. Die weitere Abtrennung der sauerstoffhaltigen Komponenten von den Kohlenwasserstoffen kann beispielsweise über mehrstufige Extraktion mittels Mixer-Settler-Systemen erfolgen und ist aufwendig. Es besteht daher Bedarf an einer vereinfachten Aufarbeitung, die ohne Extraktion auskommt oder bei der der mittels Extraktion zu behandelnde Mengenstrom zumindest reduziert wird.

**Beschreibung der Erfindung**

Diese Aufgabe wird durch ein Verfahren mit den Merkmalen des Anspruchs 1 gelöst. Weitere, insbesondere vorteilhafte Ausgestaltungen finden sich in den jeweiligen abhängigen Ansprüchen.

Erfindungsgemäßes Verfahren:

Verfahren zur Herstellung von Olefinen aus Oxygenaten, umfassend die folgenden Schritte:

(i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,

(ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,

(iii) Auftrennen des Stroms O oder eines aus diesem resultierenden Strom in einen Strom C4-, welcher zum überwiegenden Teil aus Kohlenwasserstoffen mit vier oder weniger Kohlenstoffatomen besteht und Olefine sowie Oxygenate umfasst, und in einen Strom C4+, welcher zum überwiegenden Teil aus Kohlenwasserstoffen mit vier oder mehr Kohlenstoffatomen besteht und Olefine umfasst,

(iv) Auftrennen des Stroms C4- in einen Strom C3-, der Ethylen, Propylen und Propan umfasst, und in einen Strom C4-O, der C4 Kohlenwasserstoffe und Oxygenate umfasst,

(v) Auftrennen des Stroms C3- in einen Strom C2-, der Ethylen enthält, und einen Strom C3, der Propylen und Propan umfasst, wobei der Strom C3- vor dem Auftrennen optional getrocknet wird,

(vi) Auftrennen des Stroms C3 in einen Propylen umfassenden Strom und in einen Propan umfassenden Strom, dadurch gekennzeichnet, dass

(vii) der überwiegende Teil des Stroms C4-O ohne einen weiteren Trenn- oder Aufreinigungsschritt zum OTO-Reaktor und/oder einem optional vorhandenen und diesem vorgeschalteten Veretherungsreaktor zurückgeführt wird.

Die für die Umsetzung von Oxygenaten zu Olefinprodukten erforderlichen Oxygenatumwandlungsbedingungen sind dem Fachmann aus dem Stand der Technik, beispielsweise den eingangs erörterten Druckschriften, bekannt. Es sind diejenigen physikalisch-chemischen Bedingungen, unter denen ein messbarer, bevorzugt ein technisch relevanter Umsatz von Oxygenaten zu Olefinen erzielt wird. Notwendige Anpassungen dieser Bedingungen an die jeweiligen Betriebserfordernisse wird er auf der Grundlage von Routineversuchen vornehmen. Dabei können ggf. offenbarte, spezifische Reaktionsbedingungen als Orientierung dienen, sie sind aber in Bezug auf den Umfang der Erfindung nicht einschränkend zu verstehen.

Thermische Trennverfahren im Sinne der Erfindung sind alle Trennverfahren, die auf der Einstellung eines thermodynamischen Phasengleichgewichtes beruhen. Bevorzugt sind die die Destillation oder Rektifikation. Grundsätzlich ist aber auch der Einsatz anderer thermischer Trennverfahren denkbar, beispielsweise der Extraktion oder Extraktivdestillation.

Als Aufreinigungsschritt sind im Zusammenhang mit der vorliegenden Erfindung grundsätzlich alle Verfahrensschritte zu betrachten, die sich eines thermischen Trennverfahrens bedienen; bevorzugt wird die Destillation oder Rektifikation verwendet.

Unter Fluidverbindung zwischen zwei Bereichen oder Anlagenteilen wird dabei jegliche Art von Verbindung verstanden, die es ermöglicht, dass ein Fluid, beispielsweise ein Reaktionsprodukt oder eine Kohlenwasserstofffraktion, von dem einen zu dem anderen der beiden Bereiche strömen kann, unbeachtlich etwaiger zwischengeschalteter Bereiche, Bauteile oder benötigter Fördermittel.

Unter einem Mittel wird eine Sache verstanden, die die Erreichung eines Zieles ermöglicht oder dabei behilflich ist. Insbesondere werden unter Mitteln zum Durchführen eines bestimmten Verfahrensschrittes alle diejenigen physischen Gegenstände verstanden, die der Fachmann in Betracht ziehen würde, um diesen Verfahrensschritt durchführen zu können. Beispielsweise wird der Fachmann als Mittel zum Einleiten oder Ausleiten eines Stoffstroms alle Transport- und Fördervorrichtungen, also z. B. Rohrleitungen, Pumpen, Verdichter, Ventile, in Betracht ziehen, die ihm aufgrund seines Fachwissens zur Durchführung dieses Verfahrensschrittes notwendig oder sinnvoll erscheinen.

Unter Oxygenaten werden grundsätzlich alle sauerstoffhaltigen Kohlenwasserstoffverbindungen verstanden, die sich unter Oxygenatumsetzungsbedingungen zu Olefinen, insbesondere zu kurzkettigen Olefinen wie Propylen, und weiteren Kohlenwasserstoffprodukten umsetzen lassen.

Als kurzkettige Olefine werden im Rahmen der vorliegenden Erfindung insbesondere die Olefine verstanden, die bei Umgebungsbedingungen gasförmig vorliegen, beispielsweise Ethylen, Propylen sowie die isomeren Butene 1-Buten, cis-2-Buten, trans-2-Buten, iso-Buten.

Als höhere Kohlenwasserstoffe werden im Rahmen der vorliegenden Erfindung insbesondere diejenigen Kohlenwasserstoffe verstanden, die bei Umgebungsbedingungen flüssig vorliegen.

Zur Bezeichnung von Kohlenwasserstofffraktionen wird folgende Nomenklatur verwendet: "Cn Fraktion" bezeichnet eine Kohlenwasserstofffraktion, die überwiegend Kohlenwasserstoffe der C-Kettenlänge n, also mit n C-Atomen, enthält. "Cn-Fraktion" bezeichnet eine Kohlenwasserstofffraktion, die überwiegend Kohlenwasserstoffe der C-Kettenlänge n sowie mit niedrigeren C-Kettenlängen enthält. "Cn+ Fraktion" bezeichnet eine Kohlenwasserstofffraktion, die überwiegend Kohlenwasserstoffe der C-Kettenlänge n sowie mit höheren C-Kettenlängen enthält. Aufgrund der verwendeten physikalischen Trennverfahren, beispielsweise der Destillation, ist die Auftrennung hinsichtlich der C-Kettenlänge nicht in der Weise zu verstehen, dass Kohlenwasserstoffe mit anderer Kettenlänge rigoros ausgeschlossen werden. So enthält eine Cn- Fraktion je nach Verfahrensbedingungen des Trennverfahrens immer noch geringe Mengen von Kohlenwasserstoffen mit einer C-Zahl größer als n.

Die genannten Aggregatzustände fest, flüssig und gasförmig bzw. dampfförmig sind immer in Bezug auf die lokalen physikalischen Bedingungen zu verstehen, die bei dem jeweiligen Verfahrensschritt oder in dem jeweiligen Anlagenteil herrschen, sofern nichts anderes angegeben ist. Im Rahmen der vorliegenden Anmeldung sind die Aggregatzustände gasförmig bzw. dampfförmig als synonym zu betrachten.

Unter einem Auftrennen eines Stoffstroms ist im Zusammenhang mit der vorliegenden Erfindung eine Aufteilung des Stroms in mindestens zwei Teilströme zu verstehen. Wenn nichts anderes angegeben wird, ist davon auszugehen, dass die stoffliche Zusammensetzung der Teilströme derjenigen des Ausgangsstroms entspricht, außer für die Fälle, bei denen dem Fachmann unmittelbar einsichtig ist, dass eine Änderung der stofflichen Zusammensetzung der Teilströme infolge der Bedingungen der Auftrennung zwangsweise auftreten muss.

Unter einer Benzinfraktion ist ein überwiegend, bevorzugt weitgehend vollständig aus höheren Kohlenwasserstoffen bestehendes, unter Umgebungsbedingungen flüssig vorliegendes Stoffgemisch zu verstehen, das geeignet sein kann, als Ottokraftstoff verwendet zu werden.

Unter dem überwiegenden Teil einer Fraktion, eines Stoffstroms etc. ist ein Anteil zu verstehen, der mengenmäßig größer ist als alle anderen jeweils für sich betrachteten Anteile. Insbesondere bei binären Gemischen oder bei der Auftrennung einer Fraktion in zwei Teile ist darunter ein Anteil von mehr als 50 Gew.-% zu verstehen, sofern in konkreten Fall nichts anderes angegeben ist.

Unter der Angabe, dass ein Stoffstrom überwiegend aus einer Komponente oder Komponentengruppe besteht, ist zu verstehen, dass der Stoffmengenanteil (Molenbruch) oder Massenanteil (Massenbruch) dieser Komponente oder Komponentengruppe mengenmäßig größer ist als alle anderen jeweils für sich betrachteten Anteile anderer Komponenten oder Komponentengruppen in dem Stoffstrom. Insbesondere bei binären Gemischen ist darunter ein Anteil von mehr als 50 % zu verstehen. Sofern im konkreten Fall nichts anderes angegeben ist, wird hierbei der Massenanteil (Massenbruch) zugrunde gelegt.

Unter der Angabe "Strom X oder ein aus diesem resultierender Strom", wird verstanden, dass der resultierende Strom, häufig mit X' bezeichnet, aus dem Strom X durch einen oder mehrere physikalische und/oder chemische Behandlungs- oder Umwandlungsschritte hervorgegangen ist. Ein Beispiel für einen physikalischen Behandlungsschritt ist die Kompression.

Unter der Kolonnenmitte einer Trennkolonne oder der Mitte eines bestimmten Bereichs einer Trennkolonne, beispielsweise des Abtriebsteils, ist diejenige Stelle zu verstehen, die der halben Zahl an theoretischen Böden (bei Füllkörperkolonnen oder Kolonnen mit strukturierten Packungen) oder praktischen Böden (bei Bodenkolonnen) der Kolonne oder des Kolonnenbereichs entspricht. Dies kann, muss aber nicht zwangsweise die geometrische Mitte der Kolonne oder des Kolonnenbereichs auf der halben Höhe sein. Entsprechend bezieht sich die Angabe der Kolonnenböden auf theoretische oder praktische Böden je nach Kolonnentyp.

Die erfindungsgemäße Ausgestaltung der Aufarbeitungssektion insbesondere zur Gewinnung der kurzkettigen Olefine, die stromabwärts des OTO-Reaktors, der Quenchstufe und einer optional vorhandenen Kompressionsstufe angeordnet ist, ist grundsätzlich für jede Art von Oxygenat zu Olefinanlage (OTO-Anlage) anwendbar, unabhängig von der Anlagengröße sowie vom spezifischen Design oder Konzept der vorgeschalteten Reaktionssektion, der Quenchsektion und der optional vorhandenen Kompressionssektion. Dabei werden gegenüber der aus dem Stand der Technik bekannten Aufarbeitung eines OTO-Reaktionsproduktes die nachfolgend erläuterten Maßnahmen durchgeführt und Vorteile erzielt.

Der im Trennschritt (iv) erhaltene Strom C4-O, der C4 Kohlenwasserstoffe und Oxygenate umfasst, wird zum überwiegenden Teil ohne einen weiteren Trenn- oder Aufreinigungsschritt zum OTO-Reaktor und/oder einem optional vorhandenen und diesem vorgeschalteten Veretherungsreaktor zurückgeführt. Auf diese Weise wird die Aufarbeitung des Sumpfproduktes der zugehörigen Trennkolonne, des Depropanizers, erheblich vereinfacht und es werden im Sumpfprodukt enthaltenen, reaktionsfähige Edukte (Oxygenate) und Produkte (Olefine) wieder zum OTO-Reaktor bzw. zu dem diesem vorgeschalteten Veretherungsreaktor zurückgeführt. Dies vereinfacht die Aufarbeitung des Depropanizer-Sumpfproduktes, die gemäß Stand der Technik die aufwendige, extraktive Trennung der Oxygenate von den sauerstofffreien Kohlenwasserstoffen beispielsweise in mehrstufigen Mixer-Settler-Anlagen umfasst.

Dieses Konzept kann noch dadurch unterstützt werden, dass der Depropanizer anders betrieben wird als im Stand der Technik vorgesehen. Der Depropanizer wird mit der C4- Fraktion als Kolonnenfeed beaufschlagt, die als Kopfprodukt des vorgeschalteten Trennschritts (iii) (Debutanizer) erhalten wurde und zum überwiegenden Teil aus Kohlenwasserstoffen mit vier oder weniger Kohlenstoffatomen besteht und Olefine sowie Oxygenate (vor allem Methanol und DME) umfasst. Zusätzlich wird der Depropanizer mit Methanol als Extraktionsmittel beaufschlagt, das in der Regel einige praktische bzw. theoretische Böden oberhalb der Zugabestelle für den Kolonnenfeed in die Trennkolonne eingeleitet wird. Auf diese Weise wird zwischen den beiden Zugabestellen eine Extraktions- bzw. Absorptionssektion für Oxygenate im Depropanizerfeed gebildet.

DME und Methanol sind die beiden hauptsächlichen Oxygenatkomponenten im Depropanizerfeed und können das Propylen-Endprodukt kontaminieren, so dass es nicht mehr spezifikationsgerecht z. B. für eine nachgeschaltete Polymerisierung ist. DME hat einen Normalsiedepunkt von - 25 °C; Isobutan, die niedrigsiedenste Komponente im Depropanizerfeed, weist einen Normalsiedepunkt von - 11 °C auf. Daher weist DME den niedrigsten Siedepunkt aller Komponenten auf, die mit dem Sumpfprodukt des Depropanizers, also mit dem Strom C4-O, abgeschieden werden. Die höhersiedenden Komponenten behindern daher die Abscheidung von DME mit dem Sumpfprodukt des Depropanizers. Die Aufgabe des Depropanizers besteht aber vor allem darin, den Oxygenatgehalt im Kopfprodukt zu minimieren, um die Oxygenate von dem Propylen-Endprodukt zu trennen, so dass der Oxygenatgehalt des Depropanizer-Kopfprodukts so gering sein muss wie möglich. Daher reichert sich DME im unteren Kolonnenbereich, also dem Abtriebsteil, des Depropanizers an und es bildet sich ein DME-Konzentrationsbauch. Hierunter wird ein Konzentrationsprofil in Richtung der Längsachse der Trennkolonne verstanden, bei dem die DME-Konzentration ein Maximum durchläuft, wobei der Maximalwert höher ist als die DME-Konzentration am Kolonnenkopf und im Kolonnensumpf.

Wenn der Depropanizer so betrieben wird, dass die Propylen-Verluste im Kolonnensumpf minimiert werden sollen, bildet sich im Abtriebsteil der Kolonne ein exzessiver DME-Konzentrationsbauch mit lokalen Konzentrationen von bis zu 50 Gew.-%. Dies führt zu einem instabilen Kolonnenbetrieb, da praktische Erfahrungen zeigen, dass dieser DME-Konzentrationsbauch seine Position entlang der Längsachse der Kolonne spontan verändern kann, was zu einer zeitlich fluktuierenden DME-Kontamination des Depropanizer-Kopfprodukts führt. Für einen stabilen Kolonnenbetrieb muss daher die DME-Anreicherung im unteren Kolonnenteil minimiert werden und es muss ein Kompromiss für die nachfolgend zusammengefassten Trennaufgaben des Depropanizers gefunden werden:

- Minimierung der Oxygenatmenge im Kopfprodukt
- Minimierung der DME-Anreicherung im Abtriebsteil
- Minimierung der Propylenverluste
- Minimierung der benötigten Menge an Methanol als Extraktionsmittel

Erfindungsgemäß wird dies dadurch erreicht, dass der Depropanizer so betrieben wird, dass DME weitgehend mit dem Sumpfprodukt aus der Trennkolonne ausgeleitet wird, so dass sich über die Längsachse der Trennkolonne ein nur geringer ausgeprägter DME-Konzentrationsbauch bildet. Dabei wird hingenommen, dass ein signifikanter Anteil an Propylen mit dem Sumpfprodukt die Trennkolonne verlässt. Dies führt auch dazu, dass die axialen Temperatur- und Konzentrationsprofile im Depropanizer flacher verlaufen, so dass die vorhandene Trennkapazität in Form theoretischer oder praktischer Böden besser für die Trennung von Komponenten mit ähnlicher Siedelage verwendet werden kann.

Im Depropanizerfeed, der als Kopfprodukt des vorgeschalteten Debutanizers erhalten wird, ist ein signifikanter Anteil an C4 Kohlenwasserstoffen und ein geringer Anteil an C5 Kohlenwasserstoffen enthalten. Diese Komponenten werden mit dem Sumpfprodukt aus dem Depropanizer ausgeleitet. Ein erhöhter Anteil an C4 und C5 Komponenten im Depropanizerfeed erhöht die Temperatur im Kolonnensumpf des Depropanizers und behindert somit die Abtrennung von DME mit dem Depropanizer-Sumpfprodukt. Daher ist es hilfreich, wenn der Debutanizer so betrieben wird, dass der überwiegende Anteil an C4 und C5 Komponenten diesen mit dem Sumpfprodukt verlassen und der Anteil an C4 und C5 Komponenten, die mit dem Kopfprodukt aus dem Debutanizer ausgeleitet werden und somit den Depropanizerfeed bilden, begrenzt wird.

**Weitere bevorzugte Ausgestaltungen der Erfindung**

Eine besondere Ausgestaltung der Erfindung ist dadurch gekennzeichnet, dass mindestens ein Anteil von 50 Gew.-%, bevorzugt mindestens ein Anteil von 70 Gew.-%, meist bevorzugt mindestens ein Anteil von 90 Gew.-%, des Stroms C4-O ohne einen weiteren Trenn- oder Aufreinigungsschritt zum OTO-Reaktor und /oder einem optional vorhandenen und diesem vorgeschalteten Veretherungsreaktor zurückgeführt wird. Je größer der Anteil des zurückgeführten Stroms C4-O ist, desto kleiner können etwaige Trenn- oder Aufreinigungsvorrichtungen, z. B. eine Extraktionsanlage, ausgelegt werden. Dies führt zur Einsparung von Investitionskosten. Gegebenenfalls kann auf die weitere Aufarbeitung mittels Trenn- oder Aufreinigungsvorrichtungen ganz verzichtet werden und der nicht zurückgeführte Anteil des Stroms C4-O als Purge- oder Spülstrom aus dem Verfahren ausgeleitet werden.

In besonderer Ausgestaltung des erfindungsgemäßen Verfahrens umfasst die weitere Aufarbeitung einen Extraktionsschritt zur Abtrennung der Oxygenate von den C4 Kohlenwasserstoffen im Strom C4-O. Die Extraktion kann dabei mittels einem an sich bekannten Extraktionsverfahren durchgeführt werden, beispielsweise in einer mehrstufigen Mixer-Settler-Anlage.

Bevorzugt wird bei dem erfindungsgemäßen Verfahren der in Schritt (ii) erhaltene Strom G oder der aus diesem resultierende Strom dem Schritt (iv) zugeführt wird. Auf diese Weise gehen keine leichten Olefine wie Ethylen und Propylen verloren, die auch nach dem Quenchen und/oder nach der Verdichtung gasförmig verbleiben und begehrte Wertprodukte des Verfahrens darstellen.

Bevorzugt wird es ferner, wenn Strom G und/oder Strom O einem bevorzugt mehrstufigen Kompressionsschritt zugeführt werden und bei diesem Kompressionsschritt ein gasförmige Kohlenwasserstoffe und Olefine umfassender Strom G', ein flüssiger, zum überwiegenden Teil aus Wasser und Oxygenaten bestehender wässriger Strom W' und ein flüssiger, zum überwiegenden Teil aus Kohlenwasserstoffen bestehender und Olefine umfassender Strom O' erhalten wird, wobei Strom G' dem Schritt (iv) zugeführt wird und Strom O' dem Schritt (iii) zugeführt wird. Auf diese Weise wird der für die nachfolgenden Trennschritte erforderliche Druck eingestellt und der Verlust an Olefinen, die nach der Verdichtung weiterhin flüssig oder gasförmig vorliegen, wird minimiert.

In einem weiteren Aspekt der Erfindung erfolgt das Auftrennen in den Schritten (iii), (iv), (v), (vi) mittels eines thermischen Trennverfahrens, bevorzugt mittels Destillation, fraktionierter Destillation, Rektifikation oder Extraktivdestillation oder Kombinationen daraus.

Besonders vorteilhaft ist es, wenn das Auftrennen des Stroms C4- in Schritt (iv) mittels Extraktivdestillation erfolgt, wobei ein Oxygenat als Extraktionsmittel zugegeben wird. Auf diese Weise ist insbesondere eine effektive und trennscharfe Abtrennung der Oxygenate, beispielsweise des nicht umgesetzten Dimethylethers oder Methanols, möglich. Vorteilhaft wird dabei Methanol als Oxygenat-Extraktionsmittel zugegeben, dass eine gute Wirkung als Extraktionsmittel aufweist und einen prozesseigenen Stoff darstellt, so dass verfahrensfremde Stoffe als Extraktionsmittel vermieden werden.

In besonderer Ausgestaltung des erfindungsgemäßen Verfahrens ist der untere Teil der Extraktivdestillationskolonne mit zwei Temperatursensoren ausgestattet, wobei der eine Temperatursensor bis zu 20 % oberhalb der Mitte des Abtriebsteils und der andere Temperatursensor bis zu 20 % unterhalb der Mitte des Abtriebsteils angebracht ist und wobei folgende Beziehung gilt:

$$(m(MeOH) / m(C3=)) \cdot (\Delta T(K) / \Delta n) > 0{,}3,$$

wobei

m(MeOH)   Massenstrom des als Extraktionsmittel zugegebenen Methanols

m(C3=)    Massenstrom Propylen im Strom C4-

$\Delta$n    Anzahl Kolonnenböden zwischen den zwei Temperatursensoren

$\Delta$T    Temperaturdifferenz in K zwischen den zwei Temperatursensoren

Untersuchungen haben gezeigt, dass bei Einhaltung dieser Beziehung ein stabiler Betrieb der Extraktivdestillationskolonne ermöglicht wird.

In einem weiteren Aspekt des erfindungsgemäßen Verfahrens gilt für den Massenstrom des zugegebenen Methanols m(MeOH) in den als Extraktivdestillationskolonne ausgestalteten Depropanizer:

$$m(MeOH) < 10 \cdot m(DME),$$

wobei

m(DME)    Massenstrom DME zum Trennschritt (iv)

Auch hier haben Untersuchungen gezeigt, dass bei Einhaltung dieser Beziehung ein stabiler Betrieb der Extraktivdestillationskolonne und des Gesamtverfahrens ermöglicht wird, wohingegen sich bei einem höheren Extraktionsmittel-Massenstrom der Aufwand für die Aufarbeitung und Rückführung des Methanols zu stark erhöht.

In besonderer Ausgestaltung des erfindungsgemäßen Verfahrens kann der Massenstrom des zugegebenen Methanols auch stark reduziert oder sogar auf Null zurückgeführt werden, wenn der in Schritt (vi) erhaltene, Propylen umfassende Strom durch ein Schutzbett geleitet wird, das ein für Oxygenate selektives Sorbens enthält. Auch auf diese Weise kann sichergestellt werden, dass die Reinheitsanforderungen für das Propylenprodukt eingehalten werden, so dass sich dieses ggf. für eine Weiterverarbeitung, beispielsweise eine Polymerisierung eignet.

Eine bevorzugte Ausgestaltung des erfindungsgemäßen Verfahrens ist dadurch gekennzeichnet, dass der OTO-Reaktor mindestens zwei, bevorzugt sechs Reaktionszonen umfasst und ihm ein Veretherungsreaktor vorgeschaltet ist, in dem Methanol zu DME verethert wird, wobei der Strom C4-O zum OTO-Reaktor zurückgeführt wird und der Massenstrom an DME, der vom Veretherungsreaktor zum OTO-Reaktor geführt wird, um den im Strom C4-O enthaltenen DME-Massenstrom reduziert wird. Auf diese Weise wird eine effiziente Verarbeitung des rückgeführten DME gewährleistet und ein thermisch stabiler Betrieb des OTO-Reaktors ermöglicht, da die Umsetzung von DME zu Olefinen im OTO-Reaktor stark exotherm verläuft.

Vorteilhaft ist es, wenn Schritt (iii) so durchgeführt wird, dass die Konzentration an C4+ Kohlenwasserstoffen im Kohlenwasserstoff-Einsatzstrom zum Trennschritt (iv) (Depropanizer) weniger als 30 Gew.-%, bevorzugt weniger als 20 Gew.-% beträgt. Ein erhöhter Anteil an C4 und C5 Komponenten im Depropanizerfeed erhöht die Temperatur im Kolonnensumpf des Depropanizers und behindert somit die Abtrennung von DME mit dem Depropanizer-Sumpfprodukt. Daher ist es hilfreich, wenn der Debutanizer so betrieben wird, dass der überwiegende Anteil an C4 und C5 Komponenten diesen mit dem Sumpfprodukt verlassen und der Anteil an C4 und C5 Komponenten, die mit dem Kopfprodukt aus dem Debutanizer ausgeleitet werden und somit den Depropanizerfeed bilden, begrenzt wird.

In einem weiteren Aspekt ist das erfindungsgemäße Verfahren dadurch gekennzeichnet, dass Schritt (iv) so durchgeführt wird, dass der Propylen-Gehalt im Sumpfprodukt der Trennkolonne zwischen 0,1 bis 5 Gew.-%, bevorzugt zwischen 0,2 und 2 Gew.-%, meist bevorzugt zwischen 0,5 und 1,5 Gew.-% beträgt. Im Gegensatz zu dem herkömmlichen, aus dem Stand der Technik bekannten Verfahren wird erfindungsgemäß ein gewisser Propylen-Verlust mit dem Austrag der Sumpfprodukte des Depropanizers zugelassen; dafür werden Oxygenate wie DME sicher über den Kolonnensumpf des Depropanizers entfernt und somit vom Kopfprodukt ferngehalten. Zudem reduziert sich der benötigte Mengenstrom des Methanol-Extraktionsmittels, er beträgt beispielsweise nur rund 50 % des bei dem Verfahren gemäß Stand der Technik.

Eine spezielle Ausgestaltung des erfindungsgemäßen Verfahrens sieht vor, dass Schritt (iv) so durchgeführt wird, dass die Kondensationstemperatur am Kopf der Trennkolonne kleiner als 55 °C beim einem Kolonnendruck von 23 bara (bar, absolut) ist. Auf diese Weise wird eine akzeptable Konzentration an Oxygenaten im Kopfprodukt des Depropanizers gewährleistet. Der Fachmann ist in der Lage, anhand der jeweils geltenden Reinheitsspezifikationen für die Olefin-Endprodukte auch bei geänderten Betriebsparametern des Depropanizers geeignete Wertepaare von Kondensationstemperatur und Kolonnendruck zu ermitteln und festzulegen, die ebenfalls eine ausreichende Produktreinheit im Hinblick auf enthaltene Oxygenate gewährleisten.

In einem besonderen Aspekt der für das erfindungsgemässe Verfahren verwendeten Anlage umfasst diese ferner Mittel zum Zuführen eines Teils des Stroms C4-O zu einer weiteren Aufarbeitung und/oder zum Ausleiten aus der Anlage als Purgestrom. Je größer der Anteil des zurückgeführten Stroms C4-O ist, desto kleiner können etwaige Trenn- oder Aufreinigungsvorrichtungen, z. B. eine Extraktionsanlage, ausgelegt werden. Dies führt zur Einsparung von Investitionskosten. Gegebenenfalls kann auf die weitere Aufarbeitung mittels Trenn- oder Aufreinigungsvorrichtungen ganz verzichtet werden und der nicht zurückgeführte Anteil des Stroms C4-O als Purge- oder Spülstrom aus dem Verfahren ausgeleitet werden.

In einem weiteren Aspekt der für das erfindungsgemässe Verfahren verwendeten Anlage umfasst diese ferner eine Extraktionsvorrichtung zur Abtrennung der Oxygenate von den C4 Kohlenwasserstoffen im Strom C4-O. Die Extraktion kann dabei mittels einem an sich bekannten Extraktionsverfahren durchgeführt werden, beispielsweise in einer mehrstufigen Mixer-Settler-Anlage.

In einem weiteren Aspekt der für das erfindungsgemässe Verfahren verwendeten Anlage umfasst diese ferner Mittel zum Zuführen des in der Quenchstufe erhaltenen Stroms G oder des aus diesem resultierenden Stroms zu der zweiten Trennvorrichtung. Auf diese Weise gehen keine leichten Olefine wie Ethylen und Propylen verloren, die auch nach dem Quenchen und/oder nach der Verdichtung gasförmig verbleiben und begehrte Wertprodukte des Verfahrens darstellen.

Ein weiterer Aspekt der für das erfindungsgemässe Verfahren verwendeten Anlage ist dadurch gekennzeichnet, dass die zweite Trennvorrichtung als Extraktivdestillationskolonne ausgestaltet ist, geeignet zum Betrieb mit einem Oxygenat, bevorzugt Methanol, als Extraktionsmittel. Auf diese Weise ist insbesondere eine effektive und trennscharfe Abtrennung der Oxygenate, beispielsweise des nicht umgesetzten Dimethylethers oder Methanols, möglich. Vorteilhaft wird dabei Methanol als Oxygenat-Extraktionsmittel zugegeben, dass eine gute Wirkung als Extraktionsmittel aufweist und einen prozesseigenen Stoff darstellt, so dass verfahrensfremde Stoffe als Extraktionsmittel vermieden werden.

**Ausführungsbeispiel**

Weitere Merkmale, Vorteile und Anwendungsmöglichkeiten der Erfindung ergeben sich auch aus der nachfolgenden Beschreibung der Zeichnung.

Es zeigen:

Fig. 1    eine beispielhafte Ausgestaltung eines OTO-Verfahrens bzw. einer OTO-Anlage gemäß dem Stand der Technik,

Fig. 2    eine beispielhafte Ausgestaltung eines OTO-Verfahrens bzw. einer OTO-Anlage des erfindungsgemässen Verfahrens

Fig. 3    eine beispielhafte Ausgestaltung eines OTO-Verfahrens bzw. einer OTO-Anlage des erfindungsgemässen Verfahrens mit Mess-/Regelkonzept.

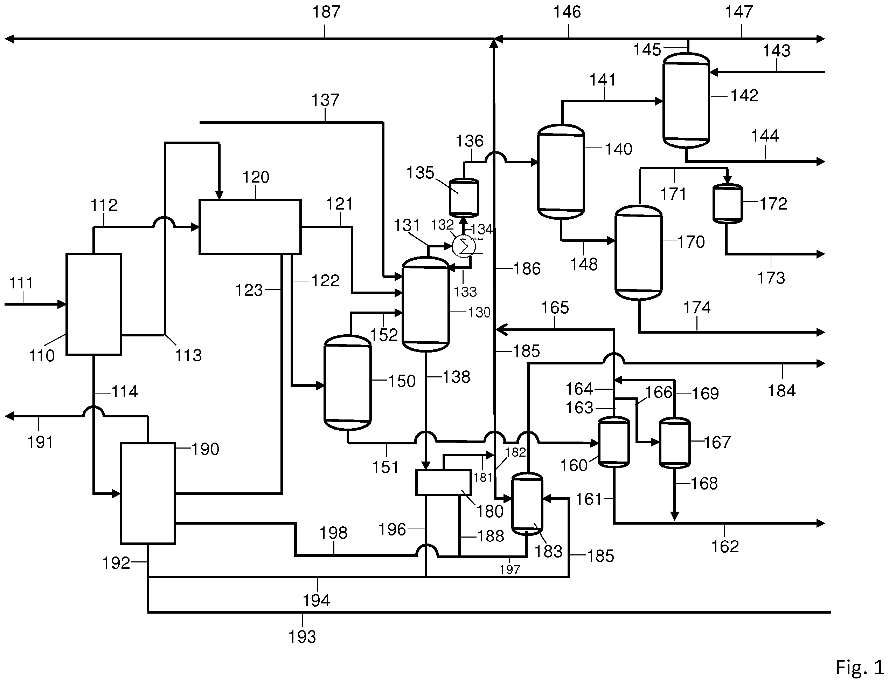

Fig. 1 zeigt eine Aufreinigung des Produktes in einem Verfahren zur Herstellung von Olefinen aus Oxygenaten gemäß dem Stand der Technik. Über Leitung 111 wird das Rohprodukt aus dem nicht dargestellten Reaktor, der Oxygenate in Olefine umgewandelt hat, in eine Quenchvorrichtung 110 überführt. Aus dieser Quenchvorrichtung wird ein gasförmiger Strom über Leitung 112 und ein flüssiger Strom über Leitung 113 einer Kompressionsvorrichtung 120 zugeführt. In dieser Kompressionsvorrichtung fällt ein gasförmiger und ein flüssiger Strom an, welche über Leitung 121 bzw. über Leitung 122 den als Rektifikationskolonnen ausgestalteten Trennkolonnen 130 bzw. 150 zugeführt werden.

Aus der Quenchvorrichtung 110 gelangt zudem eine organische flüssige Phase über Leitung 113 ebenfalls in die Kompressionsvorrichtung 120 und wird in beschriebener Weise weitergeführt. In der Rektifikationskolonne 130 (Depropanizer), die im Beispiel bei einem Druck von 23 bara betrieben wird, wird eine C3-Fraktion als Kopfprodukt von einer C4 Fraktion als Sumpfprodukt abgetrennt. Die C3- Fraktion wird am Kolonnenkopf durch Kondensation bei 55 °C erhalten und sodann über Leitung 131 einem Wärmetauscher 132 zugeführt. Über Leitung 134 gelangt ein Teil der abgekühlten C3- Fraktion in den Trockner 135, während der verbliebene Anteil der C3- Fraktion über Leitung 133 als Rücklauf in die Kolonne 130 zurückgeführt wird. Üblicherweise ist die Rektifikationskolonne 130 als Kolonne für eine Extraktivrektifikation ausgestaltet; daher wird über Leitung 137 ein Extraktionsmittel, vorzugsweise ein in dem Verfahren bereits enthaltener Stoff, deshalb besonders bevorzugt Methanol, der Kolonne aufgegeben. Das gebrauchte Extraktionsmittel wird über Leitung 138 mit den Sumpfprodukten aus der Kolonne 130 ausgeleitet.

Der flüssige Strom aus der Kompressionsstufe 120 gelangt über Leitung 122 in eine Trennkolonne 150 (Debutanizer), in welcher eine C4- Fraktion, die auch nicht umgesetzte Oxygenate, z. B. DME, enthält, als Kopfprodukt von einer C4+ Fraktion als Sumpfprodukt abgetrennt wird. Über Leitung 152 gelangt das Kopfprodukt der Trennkolonne 150 in die bereits beschriebene Trennkolonne 130, so dass hier die C3- Fraktion abgetrennt werden kann. Das Sumpfprodukt der Trennkolonne 150 wird über Leitung 151 abgeführt.

Aus dem bereits beschriebenen Trockner 135 gelangt der die kurzkettigen Olefine Ethylen und Propylen als Wertprodukte enthaltene C3- Strom in eine weitere Trennkolonne 140 (Deethanizer), in welcher eine C2- Fraktion als Kopfprodukt von einer C3-Fraktion als Sumpfprodukt abtrennt wird. Die C2- Fraktion kann über Leitung 141 entweder direkt aus dem Verfahren ausgeleitet und/oder teilweise zum OTO-Reaktor zurückgeführt werden. Alternativ kann wie dargestellt die C2-Fraktion über Leitung 141 einem Wäscher 142 zugeführt werden, der bevorzugt mit einem Waschmittel aus Wasser und NaOH betrieben wird. Das Waschmittel wird dabei über Leitung 143 zugeführt und über Leitung 144 wieder abgeführt. Das gereinigte C2- Produkt wird über Leitung 145 in Leitung 147 und 146 geleitet. Über Leitung 147 kann der C2- Strom aus dem Verfahren ausgeleitet und einer weiteren Aufarbeitung zur Gewinnung von Ethylen als Reinprodukt zugeführt werden. Über Leitungen 146 und 187 kann ein Teil des C2- Stroms zum OTO-Reaktor zurückgeführt werden.

Das Sumpfprodukt der Kolonne 140 wird über Leitung 148 in eine Trennkolonne 170 überführt. In dieser als C3 Splitter bezeichneten Trennvorrichtung 170 wird Propylen über Leitung 171 als Kopfprodukt abgezogen und kann optional einer weiteren Reinigungsvorrichtung 172 zugeführt werden, die mit einem für Oxygenate selektiven, im Handel erhältlichen Ad- bzw. Absorptionsmittel gefüllt ist und somit den Oxygenatgehalt im Propylen-Reinprodukt bis auf Spuren reduziert. Sodann wird das Propylen-Reinprodukt über Leitung 173 abgezogen und optional einer nicht bildlich nicht dargestellten Weiterverarbeitung, beispielsweise einer Polymerisierung, zugeführt werden kann. Über Leitung 174 kann das Propan enthaltende Sumpfprodukt beispielsweise in Form von Flüssiggas aus der Trennvorrichtung 170 abgezogen werden.

Das Sumpfprodukt der Trennvorrichtung 130 wird über Leitung 138 einem Mixer-Settler-Extraktionssystem 180 zugeführt. Aus diesem gelangt über Leitung 181 und Leitung 182 eine organische Phase in eine Trennvorrichtung 183, welche vorzugsweise als Extraktion ausgestaltet ist. Aus diesem wird über Leitung 184 ein C4 Kohlenwasserstoffe enthaltener Strom abgezogen.

Über Leitung 188 wird eine wässrige Fraktion aus dem Extraktionssystem 180 abgezogen. Diese gelangt über Leitung 198 in eine Methanolrückgewinnungsvorrichtung 190. In der Methanolrückgewinnungsvorrichtung 190 wird zum einen zurückgewonnenes Methanol über Leitung 191 ausgeleitet nach Umwandlung in einem nicht dargestellten Veretherungsreaktor als Oxygenat zurück in den OTO-Reaktor eingebracht. Über Leitung 192 und Leitung 133 wird Wasser aus dem System ausgeschleust. Von der Leitung 192 zweigt zudem die Leitung 194 ab. Über diese wird zum einen die Leitung 196 und damit das Mixer-Settler-Extraktionssystem mit Wasser beaufschlagt, zum anderen wird über Leitung 185 das Wasser als Extraktionsmittel in den Abscheider 183 eingebracht. In letzterem wird zudem Abwasser der Leitung 198 zugemischt und gelangt so in die Methanolrückgewinnungsvorrichtung 190.

Zudem wird über Leitung 114 die in der Quenchvorrichtung 110 gewonnene wässrige Phase und über Leitung 123 die in der Kompressionsvorrichtung 120 gewonnene wässrige Phase in die Methanolrückgewinnungsvorrichtung 190 eingebracht.

Die Trennkolonne 150 (Debutanizer) führt ihr Sumpfprodukt über Leitung 151 in die Trennvorrichtung 160 (Dehexanizer). In dieser wird eine C5+ Kohlenwasserstofffraktion über Leitung 161, 162 aus dem Sumpf als Benzinprodukt abgeführt. Eine C6- Fraktion wird über Kopf abgezogen und teilweise über Leitungen 163, 164, 165 der Leitung 186 zugemischt, welche dann über Leitung 187 Kohlenwasserstoffe, insbesondere Olefine, zurück in den OTO-Reaktor führt. Ein weiterer Teil des Kopfproduktes kann über Leitung 166 in eine Stabilisatorkolonne 167 geführt werden. Deren Sumpfprodukt wird über Leitung 168 dem Benzinprodukt zugemischt, während über Kopf mittels Leitung 169 ein im wesentlichen C4- Kohlenwasserstoffe enthaltender Strom in die Leitung 164, 165 eingebracht wird.

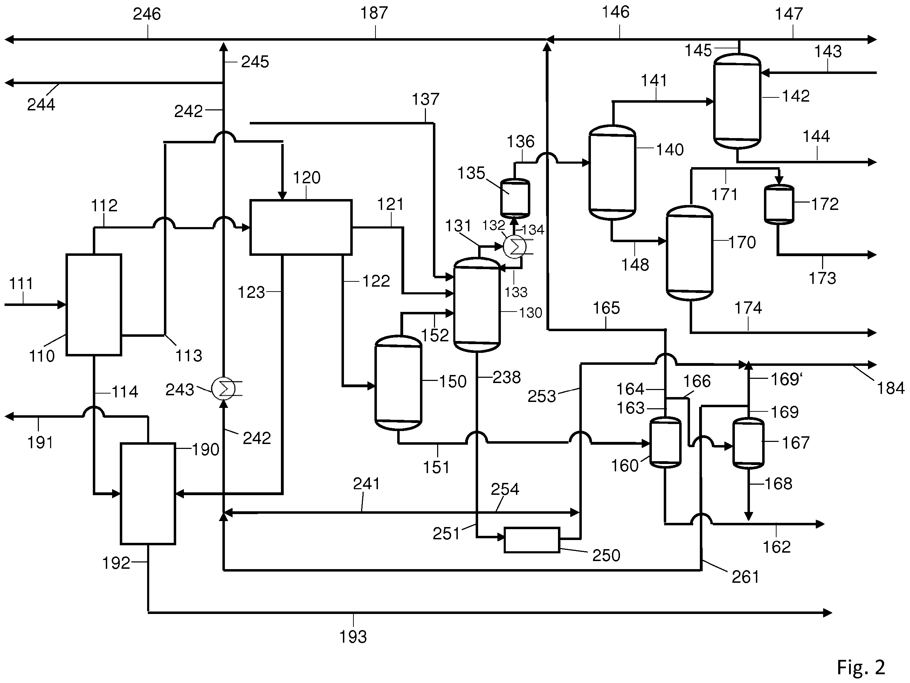

Fig. 2 zeigt ein Reinigungssystem in einem Verfahren zur Herstellung von Olefinen aus Oxygenaten in einer ersten erfindungsgemäßen Ausgestaltung. Dabei entsprechen Quenchsystem 110, Kompressionsstufe 120, Trennvorrichtung 150 (Debutanizer) sowie die Methanolrückgewinnung 190 dem aus dem Stand der Technik bekannten Aufbau. Auch die Auftrennung der C3- Fraktion in der Trennvorrichtung 140 (Deethanizer), Trennkolonne 170 (C3 Splitter) und den sich jeweils anschließenden Reinigungsvorrichtungen 142 bzw. 172 ist an sich aus dem Stand der Technik bekannt.

Durch die erfindungsgemäße Steuerung des Extraktionsmittels, welches über Leitung 137 in die Trennvorrichtung 130 (Depropanizer) geführt wird, verschiebt sich die Zusammensetzung des Sumpfproduktes in Leitung 238. Gegenüber der Zusammensetzung des Sumpfproduktes, das im Sumpf des Depropanizers im Rahmen des aus dem Stand der Technik bekannten Verfahrens erhalten wird, enthält der Sumpf der Trennkolonne 130 nunmehr sämtliche in die Kolonne eingeleiteten Oxygenate bis auf kleinste Spuren. Dabei wird in Kauf genommen, dass mehr des Wertprodukts Propylen die Trennkolonne über den Sumpf verlässt als bei dem Verfahren gemäß Stand der Technik und somit zunächst als Wertprodukt verloren geht. So beträgt der Propylen-Gehalt im Sumpfprodukt der Trennkolonne im vorliegenden Beispiel rund 1 Gew.-%. Dagegen ist er nach dem Verfahren gemäß Stand der Technik deutlich geringer und liegt beispielsweise unter 0,1 Gew.-%. Allerdings wird dieser Propylenanteil über verschiedene Wege zum OTO-Reaktor zurückgeführt und gelangt somit wieder in den Aufarbeitungsweg der Zielprodukte.

Über Leitungen 238, 241, 242, 244, 245, 246 und Wärmetauscher 243 gelangt der überwiegende Teil des Stroms C4-O, der erfindungsgemäß alle Oxygenate bis auf Spuren sowie einen signifikanten Propylenanteil umfasst, ohne einen weiteren Trenn- oder Aufreinigungsschritt zum OTO-Reaktor (Leitung 246) bzw. einem dem OTO-Reaktor vorgeschalteten Veretherungsreaktor zum Umwandlung von Methanol in DME (Leitung 244) (beide Reaktoren bildlich nicht dargestellt).

Der verbleibende, deutlich kleinere Anteil des Stroms C4-O kann optional über Leitung 251 in eine Extraktionsstufe 250 geführt werden. Diese kann erheblich kleiner ausgestaltet werden als das aus dem Stand der Technik bekannte Mixer-Settler-System. Nach der Extraktion wird der gewonnene Strom über Leitung 253 und 184 aus dem Verfahren ausgeleitet und beispielsweise einer Aufarbeitung der C4 Kohlenwasserstofffraktion zugeführt. Die in der Extraktionsstufe 250 abgetrennten Oxygenate werden über einen bildlich nicht dargestellten Leitungsweg zum OTO-Reaktor zurückgeführt.

Optional ist es möglich, aus Leitung 238 auch einen Strom abzuzweigen, der über Leitung 254 an der Extraktionsstufe 250 vorbeigeführt und direkt in Leitung 253 geführt wird. Schließlich ist es in der dargestellten erfindungsgemäßen Ausgestaltung auch möglich, über Leitung 261 einen Teilstrom des Kopfprodukts der Stabilisatorkolonne 167 über Leitung 169 abzuziehen und diesen ebenfalls teilweise zu den Reaktoren zurückzuführen. Hierzu führt Leitung 261 in Leitung 242.

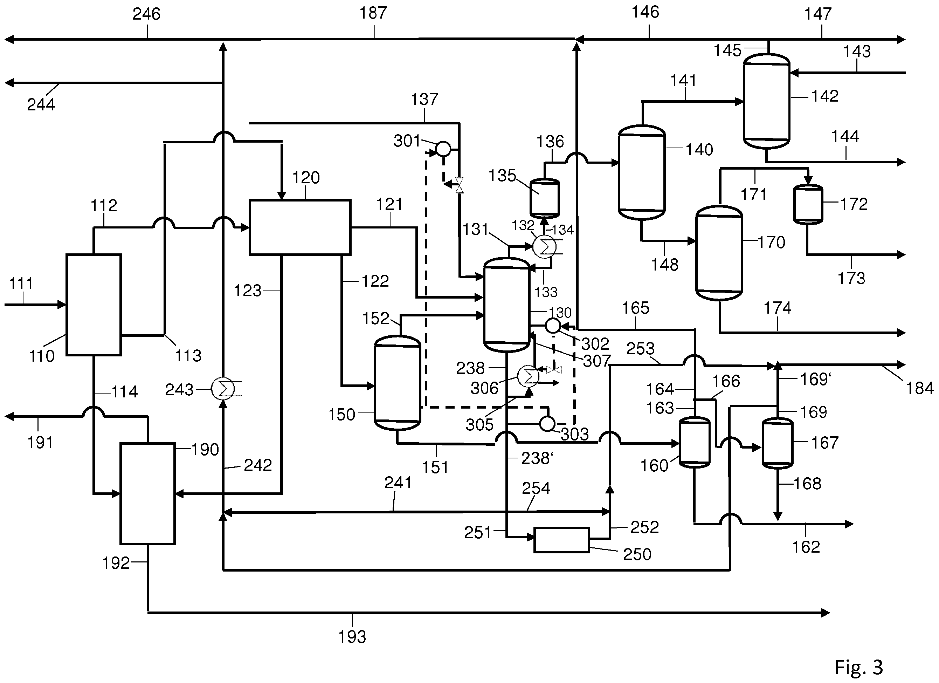

Fig. 3 zeigt die Ausgestaltung eines Reinigungssystems in einem erfindungsgemässen Verfahren zur Herstellung von Olefinen aus Oxygenaten inklusive des Steuerungs- bzw. Regelkonzeptes. In der Regelung/Steuerung 301 wird der Zufluss des Extraktionsmittels Methanol in die Kolonne 130 gesteuert oder geregelt. Dies geschieht abhängig von der über Regelungs-/Steuerungseinrichtung 302 gemessenen Temperatur innerhalb der Trennvorrichtung 130. Diese Temperatur kann über das Rücklaufverhältnis in dem Kreislauf aus Leitung 238, Leitung 305, Wärmetauscher 306 und Leitung 307 zusätzlich beeinflusst werden. Schließlich wird in der Vorrichtung 303 der Propylengehalt in Leitung 238 und somit dem Sumpf der Trennvorrichtung 130 gemessen und entsprechend die Menge des Extraktionsmittels und die Temperatur in der Kolonne gesteuert oder geregelt.

**Bezugszeichenliste**

| | |
|---|---|
| 110 | Quenchvorrichtung |
| 111 - 113 | Leitung |
| 120 | Kompressionsvorrichtung |
| 121-123 | Leitung |
| 130 | Trennkolonne (Depropanizer) |
| 131 | Leitung |
| 132 | Wärmetauscher |
| 133 | Leitung |
| 134 | Leitung |
| 135 | Trockner |
| 136, 138 | Leitung |
| 140 | Trennkolonne (Deethanizer) |
| 141 | Leitung |
| 142 | Wäscher |
| 143-148 | Leitung |
| 150 | Trennkolonne (Debutanizer) |
| 151, 152 | Leitung |
| 160 | Trennvorrichtung (Dehexanizer) |
| 161-166 | Leitung |
| 167 | Stabilisatorkolonne |
| 168, 169 | Leitung |
| 170 | Trennkolonne (C3 Splitter) |
| 171 | Leitung |
| 172 | Reinigungsvorrichtung |
| 173, 174 | Leitung |
| 180 | Mixer-Settler-Extraktionssystem |
| 181, 182 | Leitung |
| 183 | Abscheider |
| 184-187 | Leitung |
| 190 | Methanolrückgewinnungsvorrichtung |
| 191-198 | Leitung |
| | |
| 238, 241, 242 | Leitung |
| 243 | Wärmetauscher |
| 244-246 | Leitung |
| 250 | Extraktionsstufe |
| 251, 253 | Leitung |
| 261 | Leitung |

| 301 | Regelung/Steuerung |
|---|---|
| 302 | Regelung/Steuerung |
| 303 | Regelung/Steuerung |
| 305 | Leitung |
| 306 | Wärmetauscher |
| 307 | Leitung |

**Patentansprüche**

1. Verfahren zur Herstellung von Olefinen aus Oxygenaten, umfassend die folgenden Schritte:

   (i) Heterogen-katalysierte Umsetzung der Oxygenate in einem Oxygenat-zu-Olefin-Reaktor (OTO-Reaktor) unter Oxygenatumwandlungsbedingungen zu einem Produktstrom, der Wasser, Olefine und andere Kohlenwasserstoffe und wenigstens ein Oxygenat enthält,
   (ii) Quenchen des Produktstroms in wenigstens einer Quenchstufe, wodurch ein gasförmige Kohlenwasserstoffe und Olefine umfassender Strom G, ein flüssiger, zum überwiegenden Teil aus Wasser und Oxygenaten bestehender wässriger Strom W und ein flüssiger, zum überwiegenden Teil aus Kohlenwasserstoffen bestehender und Olefine umfassender Strom O erhalten wird,
   (iii) Auftrennen des Stroms O oder eines aus diesem resultierenden Strom in einen Strom C4-, welcher zum überwiegenden Teil aus Kohlenwasserstoffen mit vier oder weniger Kohlenstoffatomen besteht und Olefine sowie Oxygenate umfasst, und in einen Strom C4+, welcher zum überwiegenden Teil aus Kohlenwasserstoffen mit vier oder mehr Kohlenstoffatomen besteht,
   (iv) Auftrennen des Stroms C4- in einen Strom C3-, der Ethylen, Propylen und Propan umfasst, und in einen Strom C4-O, der C4 Kohlenwasserstoffe und Oxygenate umfasst,
   (v) Auftrennen des Stroms C3- in einen Strom C2-, der Ethylen enthält, und einen Strom C3, der Propylen und Propan umfasst, wobei der Strom C3- vor dem Auftrennen optional getrocknet wird,
   (vi) Auftrennen des Stroms C3 in einen Propylen umfassenden Strom und in einen Propan umfassenden Strom, **dadurch gekennzeichnet, dass**
   (vii) der überwiegende Teil des Stroms C4-O ohne einen weiteren Trenn- oder Aufreinigungsschritt zum OTO-Reaktor und/oder einem optional vorhandenen und diesem vorgeschalteten Veretherungsreaktor zurückgeführt wird.

2. Verfahren nach Anspruch 1, **dadurch gekennzeichnet, dass** mindestens ein Anteil von 50 Gew.-%, bevorzugt mindestens ein Anteil von 70 Gew.-%, meist bevorzugt mindestens ein Anteil von 90 Gew.-%, des Stroms C4-O ohne einen weiteren Trenn- oder Aufreinigungsschritt zum OTO-Reaktor und/oder einem optional vorhandenen und diesem vorgeschalteten Veretherungsreaktor zurückgeführt wird.

3. Verfahren nach Anspruch 1 oder 2, **dadurch gekennzeichnet, dass** der verbleibende Anteil des Stroms C4-O einer weiteren Aufarbeitung zugeführt und/ oder als Purgestrom aus dem Verfahren ausgeleitet wird.

4. Verfahren nach Anspruch 3, **dadurch gekennzeichnet, dass** die weitere Aufarbeitung einen Extraktionsschritt zur Abtrennung der Oxygenate von den C4 Kohlenwasserstoffen im Strom C4-O umfasst,

5. Verfahren nach einem der vorigen Ansprüche, **dadurch gekennzeichnet, dass** der in Schritt (ii) erhaltene Strom G oder der aus diesem resultierende Strom dem Schritt (iv) zugeführt wird.

6. Verfahren nach einem der vorigen Ansprüche, **dadurch gekennzeichnet, dass** Strom G und/oder Strom O einem bevorzugt mehrstufigen Kompressionsschritt zugeführt werden und bei diesem Kompressionsschritt ein gasförmige Kohlenwasserstoffe und Olefine umfassender Strom G', ein flüssiger, zum überwiegenden Teil aus Wasser und Oxygenaten bestehender wässriger Strom W' und ein flüssiger, zum überwiegenden Teil aus Kohlenwasserstoffen bestehender und Olefine umfassender Strom O' erhalten wird, wobei Strom G' dem Schritt (iv) zugeführt wird und Strom O' dem Schritt (iii) zugeführt wird.

7. Verfahren nach einem der vorigen Ansprüche, **dadurch gekennzeichnet, dass** die Trennschritte (iii), (iv), (v), (vi) mittels eines thermischen Trennverfahrens, bevorzugt mittels Destillation, fraktionierter Destillation, Rektifikation oder Extraktivdestillation oder Kombinationen daraus erfolgt.

**8.** Verfahren nach Anspruch 7, **dadurch gekennzeichnet, dass** das Auftrennen in Schritt (iv) mittels Extraktivdestillation in einer Extraktivdestillationskolonne erfolgt, wobei ein Oxygenat, bevorzugt Methanol, als Extraktionsmittel zugegeben wird.

**9.** Verfahren nach Anspruch 8, **dadurch gekennzeichnet, dass** die Extraktivdestillationskolonne mit zwei Temperatursensoren ausgestattet ist, wobei der eine Temperatursensor bis zu 20 % oberhalb der Kolonnenmitte und der andere Temperatursensor bis zu 20 % unterhalb der Kolonnenmitte angebracht ist und wobei folgende Beziehung gilt:

$$(m(MeOH) / m(C3=)) * (\Delta T(K) / \Delta n) > 0,3,$$

wobei

m(MeOH ) Massenstrom des als Extraktionsmittel zugegebenen Methanols
m(C3=) Massenstrom Propylen im Strom C4-
Δn Anzahl Kolonnenböden zwischen den zwei Temperatursensoren
ΔT Temperaturdifferenz in K zwischen den zwei Temperatursensoren

**10.** Verfahren nach Anspruch 8 oder 9, **dadurch gekennzeichnet, dass** für den Massenstrom des zugegebenen Methanols m(MeOH) gilt:

$$m(MeOH) < 10 * m(DME),$$

wobei

m(DME) Massenstrom DME zum Trennschritt (iv)

**11.** Verfahren nach einem der Ansprüche 7 bis 10, **dadurch gekennzeichnet, dass** der Massenstrom des zugegebenen Methanols m(MeOH) Null oder nahe Null beträgt und dass der in Schritt (vi) erhaltene, Propylen umfassende Strom durch ein Schutzbett geleitet wird, das ein für Oxygenate selektives Sorbens enthält.

**12.** Verfahren nach einem der vorigen Ansprüche, **dadurch gekennzeichnet, dass** der OTO-Reaktor mindestens zwei, bevorzugt sechs Reaktionszonen umfasst und ihm ein Veretherungsreaktor vorgeschaltet ist, in dem Methanol zu DME verethert wird, wobei der Strom C4-O zum OTO-Reaktor zurückgeführt wird und der Massenstrom an DME, der vom Veretherungsreaktor zum OTO-Reaktor geführt wird, um den im Strom C4-O enthaltenen DME-Massenstrom reduziert wird.

**13.** Verfahren nach einem der vorigen Ansprüche, **dadurch gekennzeichnet, dass** Schritt (iii) so durchgeführt wird, dass die Konzentration an C4+ Kohlenwasserstoffen im Kohlenwasserstoff-Einsatzstrom zum Trennschritt (iv) (Depropanizer) weniger als 30 Gew.-%, bevorzugt weniger als 20 Gew.-% beträgt.

**14.** Verfahren nach einem der vorigen Ansprüche, **dadurch gekennzeichnet, dass** Schritt (iv) so durchgeführt wird, dass der Propylen-Gehalt im Sumpfprodukt der Trennkolonne zwischen 0,1 bis 5 Gew.-%, bevorzugt zwischen 0,2 und 2 Gew.-%, meist bevorzugt zwischen 0,5 und 1,5 Gew.-% beträgt.

**15.** Verfahren nach einem der vorigen Ansprüche, **dadurch gekennzeichnet, dass** Schritt (iv) so durchgeführt wird, dass die Kondensationstemperatur am Kopf der Trennkolonne kleiner als 55 °C beim einem Kolonnendruck von 23 bara ist.

**Claims**

**1.** Process for preparing olefins from oxygenates, comprising the following steps:

(i) heterogeneously catalysed conversion of the oxygenates in an oxygenate-to-olefin reactor (OTO reactor) under oxygenate conversion conditions to give a product stream containing water, olefins and other hydrocarbons and at least one oxygenate,

(ii) quenching the product stream in at least one quench stage to obtain a gaseous stream G comprising hydrocarbons and olefins, a liquid aqueous stream W consisting predominantly of water and oxygenates, and a liquid stream Q consisting predominantly of hydrocarbons and comprising olefins,

(iii) separating the stream O or a stream resulting therefrom into a stream C4- consisting predominantly of hydrocarbons having four or fewer carbon atoms and comprising olefins and oxygenates, and into a stream C4+ consisting predominantly of hydrocarbons having four or more carbon atoms,

(iv) separating the stream C4- into a stream C3-comprising ethylene, propylene and propane and into a stream C4-O comprising C4 hydrocarbons and oxygenates,

(v) separating the stream C3- into a stream C2-containing ethylene and a stream C3 comprising propylene and propane, where the stream C3- is optionally dried prior to the separating,

(vi) separating the stream C3 into a stream comprising propylene and into a stream comprising propane, **characterized in that**

(vii) the predominant portion of the stream C4-O is returned without any further separation or purification step to the OTO reactor and/or to an optionally present etherification reactor upstream thereof.

2. Process according to Claim 1, **characterized in that** at least a proportion of 50% by weight, preferably at least a proportion of 70% by weight, most preferably at least a proportion of 90% by weight, of the stream C4-O is returned without any further separation or purification step to the OTO reactor and/or to an optionally present etherification reactor upstream thereof.

3. Process according to Claim 1 or 2, **characterized in that** the remaining proportion of the stream C4-O is sent to a further workup and/or discharged from the process as a purge stream.

4. Process according to Claim 3, **characterized in that** the further workup comprises an extraction step for separation of the oxygenates from the C4 hydrocarbons in the stream C4-O.

5. Process according to any of the preceding claims, **characterized in that** the stream G obtained in step (ii) or the stream resulting therefrom is sent to step (iv).

6. Process according to any of the preceding claims, **characterized in that** stream G and/or stream O is sent to a preferably multistage compression step and, in this compression step, a gaseous stream G' comprising hydrocarbons and olefins, a liquid aqueous stream W' consisting predominantly of water and oxygenates, and a liquid stream O' consisting predominantly of hydrocarbons and comprising olefins are obtained, where stream G' is sent to step (iv) and stream O' is sent to step (iii).

7. Process according to any of the preceding claims, **characterized in that** the separation steps (iii), (iv), (v), (vi) are effected by means of a thermal separation process, preferably by means of distillation, fractional distillation, rectification or extractive distillation or combinations thereof.

8. Process according to Claim 7, **characterized in that** the separating in step (iv) is effected by means of extractive distillation in an extractive distillation column, with addition of an oxygenate, preferably methanol, as extractant.

9. Process according to Claim 8, **characterized in that** the extractive distillation column is equipped with two temperature sensors, where one temperature sensor is mounted up to 20% above the middle of the column and the other temperature sensor up to 20% below the middle of the column and where the following relationship applies:

$$(m(MeOH) \ / \ m(C3=)) \ \bullet \ (\Delta T(K) \ / \ \Delta n) \ > \ 0.3$$

where

m(MeOH) mass flow rate of the methanol added as extractant
m(C3=) mass flow rate of propylene in the stream C4-$\Delta n$ number of column trays between the two temperature sensors
$\Delta T$ temperature differential in K between the two temperature sensors.

10. Process according to Claim 8 or 9, **characterized in that** the mass flow rate of the methanol added m(MeOH) is subject to the following condition:

$$m(MeOH) < 10 \cdot m(DME)$$

where

m(DME) mass flow rate of DME to separation step (iv).

11. Process according to any of Claims 7 to 10, **characterized in that** the mass flow rate of the methanol added m(MeOH) is zero or close to zero, and **in that** the propylene-comprising stream obtained in step (vi) is guided through a guard bed containing a sorbent selective for oxygenates.

12. Process according to any of the preceding claims, **characterized in that** the OTO reactor comprises at least two, preferably six, reaction zones, and it is connected downstream of an etherification reactor in which methanol is etherified to give DME, where the stream C4-O is recycled to the OTO reactor and the mass flow rate of DME which is conducted from the etherification reactor to the OTO reactor is reduced by the DME mass flow rate present in the stream C4-O.

13. Process according to any of the preceding claims, **characterized in that** step (iii) is conducted in such a way that the concentration of C4+ hydrocarbons in the hydrocarbon feed stream to the separation step (iv) (depropanizer) is less than 30% by weight, preferably less than 20% by weight.

14. Process according to any of the preceding claims, **characterized in that** step (iv) is conducted in such a way that the propylene content in the bottom product from the separation column is between 0.1% and 5% by weight, preferably between 0.2% and 2% by weight, most preferably between 0.5% and 1.5% by weight.

15. Process according to any of the preceding claims, **characterized in that** step (iv) is conducted in such a way that the condensation temperature at the top of the separation column is less than 55°C at a column pressure of 23 bara.

**Revendications**

1. Procédé pour la production d'oléfines à partir de composés oxygénés, comprenant les étapes suivantes :

(i) mise en réaction, à catalyse hétérogène, des composés oxygénés dans un réacteur de conversion de composés oxygénés en oléfines (réacteur OTO) dans des conditions de conversion de composés oxygénés, conduisant à un courant de produit qui contient de l'eau, des oléfines et d'autres hydrocarbures et au moins un composé oxygéné,
(ii) refroidissement du courant de produit dans au moins un étage de refroidissement, ce par quoi on obtient un courant G comprenant des oléfines et hydrocarbures gazeux, un courant W liquide aqueux constitué en majeure partie d'eau et de composés oxygénés et un courant O liquide constitué en majeure partie d'hydrocarbures et comprenant des oléfines,
(iii) fractionnement du courant O ou d'un courant résultant de celui-ci en un courant C4- qui est constitué en majeure partie d'hydrocarbures ayant quatre ou moins de quatre atomes de carbone et comprend des oléfines ainsi que des composés oxygénés, et en un courant C4+ qui est constitué en majeure partie d'hydrocarbures ayant quatre ou plus de quatre atomes de carbone,
(iv) fractionnement du courant C4- en un courant C3-qui comprend de l'éthylène, du propylène et du propane, et en un courant C4-O qui comprend des hydrocarbures en C4 et des composés oxygénés,
(v) fractionnement du courant C3- en un courant C2-qui contient de l'éthylène, et un courant C3 qui comprend du propylène et du propane, le courant C3-étant en option séché avant le fractionnement,
(vi) fractionnement du courant C3 en un courant comprenant du propylène et en un courant comprenant du propane,
**caractérisé en ce que**
(vii) la majeure partie du courant C4-O est renvoyé, sans une étape ultérieure de fractionnement ou de purification, au réacteur OTO et/ou à un réacteur d'éthérification présent en option et raccordé en amont à celui-ci,

2. Procédé selon la revendication 1, **caractérisé en ce qu'**au moins une proportion de 50 % en poids, de préférence au moins une proportion de 70% en poids, de façon tout particulièrement préférée au moins une proportion de 90 % en poids du courant C4-O est renvoyée, sans une étape ultérieure de fractionnement ou de purification, au réacteur OTO et/ou à un réacteur d'éthérification présent en option et raccordé en amont à celui-ci.

3. Procédé selon la revendication 1 ou 2, **caractérisé en ce que** la: partie restante du courant C4-O est envoyée à un traitement final ultérieur et/ou évacuée du processus en tant que courant de purge.

4. Procédé selon la revendication 3, **caractérisé en ce que** le traitement final ultérieur comprend une étape d'extraction destinée à la séparation des composés oxygénés d'avec les hydrocarbures en C4 dans le courant C4-O.

5. Procédé selon l'une quelconque des revendications précédentes, **caractérisé en ce que** le courant G obtenu dans l'étape (ii) ou le courant résultant de celui-ci est envoyé à l'étape (iv).

6. Procédé selon l'une quelconque des revendications précédentes, **caractérisé en ce que** le courant G et/ou le courant O est/sont envoyé (s) à une étape de compression de préférence à plusieurs étages et dans cette étape dé compression on obtient un courant G' comprenant des oléfines et hydrocarbures gazeux, un courant W' liquide aqueux constitué en majeure partie d'eau et de composés oxygénés et un courant O' liquide constitué en majeure partie d'hydrocarbures et comprenant des oléfines, le courant G' étant envoyé à l'étape (iv) et le courant O' étant envoyé à l'étape (iii).

7. Procédé selon l'une quelconque des revendications précédentes, **caractérisé en ce que** les étapes de fractionnement (iii), (iv), (v), (vi) s'effectuent par un procédé de séparation thermique, de préférence par distillation, distillation fractionnée, rectification ou distillation extractive ou des combinaisons de celles-ci.

8. Procédé selon la revendication 7, **caractérisé en ce que** le fractionnement dans l'étape (iv) s'effectue par distillation extractive dans une colonne de distillation extractive, un composé oxygéné, du méthanol de préférence, étant ajouté en tant qu'agent d'extraction.

9. Procédé selon la revendication 8, **caractérisé en ce que** la colonne de distillation extractive est munie de deux capteurs de température, un capteur de température étant placé jusqu'à 20 % au-dessus du milieu de la colonne et l'autre capteur de température étant placé jusqu'à 20 % au-dessous du milieu de la colonne et la relation suivante s'appliquant :

$$(m(MeOH) / m(C3=)) * (\Delta T(K) / \Delta n) > 0,3,$$

où

m(MeOH) flux massique du méthanol ajouté en tant qu'agent d'extraction
m(C3=) flux massique de propylène dans le courant C4-
$\Delta n$ nombre de plateaux de la colonne entre les deux capteurs de température
$\Delta T$ différence de température en K entre les deux capteurs de température.

10. Procédé selon la revendication 8 ou 9, **caractérisé en ce que** pour le flux massique du méthanol ajouté (m(MeOH) il s'applique que

$$m(MeOH) < 10 . m(DME),$$

où

m(DME) flux massique de DME arrivant à l'étape de fractionnement (iv).

11. Procédé selon l'une quelconque des revendications 7 à 10, **caractérisé en ce que** le flux massique du méthanol ajouté m(MeOH) vaut zéro ou presque zéro et **en ce que** le courant comprenant du propylène, obtenu dans l'étape (vi), est envoyé à travers un lit protecteur qui contient un sorbant sélectif pour les composés oxygénés.

12. Procédé selon l'une quelconque des revendications précédentes, **caractérisé en ce que** le réacteur OTO comprend au moins deux, de préférence six, zones de réaction et lui est raccordé en amont un réacteur d'éthérification dans lequel le méthanol est éthérifié en DME, le courant C4-O étant renvoyé au réacteur OTO et le flux massique de DME qui est conduit du réacteur d'éthérification au réacteur OTO étant réduit du flux massique de DME contenu dans le courant C4-O.

13. Procédé selon l'une quelconque des revendications précédentes, **caractérisé en ce que** l'étape (iii) est effectuée de telle façon que la concentration d'hydrocarbures en C4+ dans le courant de charge d'hydrocarbures arrivant à l'étape de séparation (iv) (dépropaniseur) est inférieure à 30 % en poids, de préférence inférieure à 20 % en poids.

14. Procédé selon l'une quelconque des revendications précédentes, **caractérisé en ce que** l'étape (iv) est effectuée de telle façon que la teneur en propylène du produit de bas de la colonne de séparation est comprise entre 0,1 et 5 % en poids, de préférence entre 0,2 et 2 % en poids, de façon tout particulièrement préférée entre 0,5 et 1,5 % en poids.

15. Procédé selon l'une quelconque des revendications précédentes, **caractérisé en ce que** l'étape (iv) est effectuée de telle façon que la température de condensation à la tête de la colonne de séparation est inférieure à 55 °C sous une pression dans la colonne de 23 bars abs.

Fig. 1

Fig. 2

Fig. 3

EP 3 683 202 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102014112792 A1
- DE 102013101575 A1